# EUROPÄISCHE PATENTSCHRIFT

(11) **EP 2 655 582 B1**
(45) Veröffentlichungstag und Bekanntmachung des Hinweises auf die Patenterteilung: **04.10.2017**
(21) Anmeldenummer: 11774016.7
(22) Anmeldetag: 17.10.2011
(51) Int. Cl.: C11B 9/00, A61K 8/49, A61Q 13/00, C09J 11/06, C11D 3/50

(54) **PARFÜMZUSAMMENSETZUNG UMFASSEND RIECHSTOFF-ALDEHYD ODER -KETON SOWIE OXAZOLIDIN-RIECHSTOFFVORLÄUFER**
PERFUME COMPOSITION COMPRISING FRAGRANCE ALDEHYDE OR KETONE AND OXAZOLIDINE FRAGRANCE PRECURSOR
COMPOSITION DE PARFUM COMPRENANT UN COMPOSANT ODORANT ALDÉHYDE OU -CÉTONE ET PRÉCURSEUR DE COMPOSANT ODORANT OXAZOLIDINE

(30) Priorität: 21.12.2010 DE 102010063693
(43) Veröffentlichungstag der Anmeldung: 30.10.2013
(73) Patentinhaber: Henkel AG & Co. KGaA, 40589 Düsseldorf (DE)
(72) Erfinder: HUCHEL, Ursula, 50670 Köln (DE); BAUER, Andreas, 41564 Kaarst (DE); BUNN, Ralf, 40223 Düsseldorf (DE); SMYREK, Hubert, 47804 Krefeld (DE); MATERNE, Manuela, 41564 Kaarst (DE); RITTLER, Frank, 40547 Düsseldorf (DE)
(74) Vertreter: Viering, Jentschura & Partner mbB Patent- und Rechtsanwälte
(86) Internationale Anmeldenummer: PCT/EP2011/068050
(87) Internationale Veröffentlichungsnummer: WO 2012/084292

(56) Entgegenhaltungen:
- WO-A1-2010/105874
- WO-A1-2010/142481
- WO-A2-2010/105873
- DE-A1-102006 003 092

## Beschreibung

Die vorliegende Erfindung betrifft spezielle Parfümzusammensetzungen. Sie betrifft ferner Verbrauchsprodukte, wie insbesondere Wasch- oder Reinigungsmittel, Kosmetika oder Klebstoffe, welche solche Parfümzusammensetzungen enthalten. Sie betrifft ferner ein Verfahren zur Beduftung von Textilien.

Bei der Parfümierung von Verbrauchsprodukten wird in der Regel so vorgegangen, dass ein Riechstoffe enthaltendes Parfumöl direkt mit dem Zielprodukt, wie z.B. einem Waschmittel, vermischt wird. Problematisch kann dabei sein, dass leichtflüchtige Riechstoffe bereits während der Einarbeitung in das Produkt oder im Verlauf der Lagerung teilweise durch Ausdampfen verloren gehen können. Auch sind zahlreiche Riechstoffe, wie insbesondere Aldehyde, unter den jeweils gegebenen Bedingungen instabil, so dass es zur teilweisen oder vollständigen Zersetzung von Riechstoffen kommen kann. Die entsprechenden Substanzen sind dann im Endprodukt zum Teil nur noch schwach oder gar nicht mehr wahrnehmbar. Dies kann z.B. zu einer Veränderung des Gesamtgeruchseindrucks der Komposition führen. Deshalb besteht das Bedürfnis, Riechstoffe zu schützen. Zusätzlich besteht das Bedürfnis, eine möglichst lange anhaltende Duftwirkung zu erhalten, beispielsweise mit Blick auf Wasch- und Reinigungsvorgänge. Für diese Zwecke werden im Stand der Technik Riechstoffvorläufer beschrieben.

Die Deutsche Auslegeschrift DE 1 133 847 beschreibt den Einsatz der Kondensationsprodukte von Aldehyden und Ketonen mit Oxiaminen in der Parfümerie an Stelle der freien Aldehyde und Ketone. Dazu werden die Aldehyde und Ketone mit Ethanolamin oder Diethanolamin umgesetzt. In US 6,861,402 sind Riechstoffvorläufer beschrieben, die einen Riechstoffaldehyd oder ein Riechstoffketon in Form eines Oxazolidins gebunden enthalten. Dabei wird beispielsweise N-Benzolethanolamin mit einem Riechstoff umgesetzt, so dass sich ein monocyclisches Oxazolidin ergibt. In US 2003/0207786 A1 sind ebenfalls Riechstoffvorläufer beschrieben, die eine Oxazolidinstruktur aufweisen. In US 4,277,353 werden mono- und bicyclische Oxazolidine als korrosionsinhibierende Additive für Schmieröle beschrieben. In US 2004/0087453 A1 werden bestimmte photolabile Riechstoffvorläufer beschrieben, welche auch in Form von Oxazolidinen gebunden sein können. In US 2004/0067870 A1 werden spezielle Riechstoff-Aldehyde mit einem tertiären alpha-Kohlenstoffatom beschrieben, welche auch in Form von Oxazolidinen gebunden sein können. In US 2003/0158079 A1 werden Wirkstoffabgabesysteme, geeignet zum Abgeben eines Wirkstoffs an ein Substrat, beschrieben, wobei das Wirkstoffabgabesystem einen Wirkstoff in der Form eines Aldehyds oder Ketons und ein Amin umfasst, das eine primäre und/oder sekundäre Amineinheit umfasst. WO2007/087977 A1 betrifft 1-Aza-3,7-dioxabicyclo[3.3.0]octan-Verbindungen, deren Einsatz zu einem verlängerten Dufteindruck von Riechstoffaldehyden und Riechstoffketonen führt. Die Lehre, bestimmte Mischungen von 1-Aza-3,7-dioxabicyclo[3.3.0]octan-Verbindungen und Aldehyhden oder Ketonen in Parfümzusammensetzungen einzusetzen, kann der WO2007/087977 allerdings nicht unmittelbar und eindeutig entnommen werden.

Aufgabe der vorliegenden Erfindung war es, eine Parfümzusammensetzung bereitzustellen, welche sowohl eine gute Produktbeduftung (z.B. eines Waschmittels) ermöglicht als auch eine lang anhaltende Duftwirkung ermöglicht.

Diese Aufgabe wird vom Gegenstand der Erfindung gelöst. Der Gegenstand der vorliegenden Erfindung ist eine Parfümzusammensetzung, umfassend
(a) zumindest einen Riechstoff-Aldehyd mit mindestens 6, vorzugsweise mindestens 7, insbesondere mindestens 8 Kohlenstoffatomen oder ein Riechstoff-Keton mit mindestens 6, vorzugsweise mindestens 7, insbesondere mindestens 8 Kohlenstoffatomen sowie
(b) zumindest einen mit dem unter (a) genannten Riechstoff-Aldehyd oder Riechstoff-Keton korrespondierenden Oxazolidin-Riechstoffvorläufer, welcher den gleichen Riechstoff-Aldehyd bzw. das gleiche Riechstoff-Keton freisetzen kann, wobei das Molverhältnis von Riechstoff-Aldehyd und/oder Riechstoff-Keton (a) zu dem korrespondierenden Oxazolidin-Riechstoffvorläufer (b) 20:1 bis 1:20, vorzugsweise 10:1 bis 1:10, vorteilhafterweise 5:1 bis 1:5 beträgt und wobei es sich bei dem Oxazolidin-Riechstoffvorläufer um eine 1-Aza-3,7-dioxabicyclo[3.3.0]octan-Verbindung der allgemeinen Formel (I) handelt, 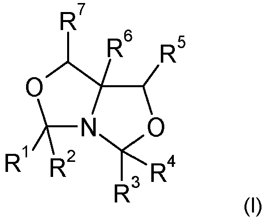 wobei
   - R¹, R², R³, R⁴: unabhängig voneinander für Reste stehen, die in einer Verbindung der all-gemeinen Formel R¹-C(=O)-R² bzw. R³-C(=O)-R⁴ einen Riechstoff-Aldehyd mit mindestens 6, vorzugsweise mindestens 7, insbesondere mindestens 8 Kohlenstoffatomen oder ein Riechstoff-Keton mit mindestens 6, vorzugsweise mindestens 7, insbesondere mindestens 8 Kohlenstoffatomen ergeben,
   - R⁵, R⁶, R⁷: unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann.

Die erfindungsgemäße Parfümzusammensetzung enthält also Mischungen aus freiem Riechstoff-Aldehyd und/oder -Keton mit mindestens 6 Kohlenstoffatomen und dem entsprechenden Oxazolidin, in welchem der gleiche Riechstoff-Aldehyd und/oder -Keton in gebundener Form vorliegt.

Es wurde überraschenderweise gefunden, dass solche Parfümzusammensetzungen eine besonders gute und effiziente Beduftung von üblichen Verbrauchsprodukten, wie insbesondere Wasch- oder Reinigungsmitteln, Kosmetika, Klebstoffen ermöglichen. Es kann mit Hilfe der erfindungsgemäßen Parfümzusammensetzungen insbesondere ein frischer Dufteindruck erzeugt werden, der auch lange anhält. Hinzukommt, dass die Einarbeitung der erfindungsgemäßen Parfümzusammensetzungen in übliche Verbrauchsprodukten zu sehr stabilen Produkten führt. Die Produkte, wie z.B. Waschmittel, sind besonders lagerstabil, sogar unter kritischeren Bedingungen, wie z.B. bei erhöhten Temperaturen, wie sie z.B. in der tropischen und subtropischen Klimazone auftreten können. Diese Vorteile wurden sowohl für feste wie für flüssige Produkte, wie insbesondere Wasch- oder Reinigungsmittel, z.B. Weichspüler, gefunden. Es wurde weiterhin überraschenderweise gefunden, dass die erfindungsgemäßen Parfümzusammensetzungen eine besonders gute und lang anhaltende Objektbeduftung bei der Anwendung der entsprechend parfümierten Verbrauchsprodukte ermöglichen, z.B. im Rahmen von Wasch- oder Reinigungsvorgängen. Insbesondere konnte in diesem Zusammenhang ein besonders lang anhaltender Duft getrockneter Wäsche gefunden werden.

Alle Kohlenwasserstoff-Reste im Sinne der Erfindung können grundsätzlich acyclisch oder cyclisch, substituiert oder unsubstituiert, verzweigt oder unverzweigt sowie gesättigt oder ungesättigt sein. Die Kohlenwasserstoff-Reste im Sinne der Erfindung können grundsätzlich Heteroatome, wie z.B. Stickstoff-, Sauerstoff- oder Schwefel-Atome umfassen. Bevorzugt mit Blick auf R⁵, R⁶ und R⁷ sind jeweils acyclische, unverzweigte Kohlenwasserstoffreste, die ggf. substituiert sein können. Geeignete Substituenten sind z.B. Hydroxy-, Alkoxy-, Amino- oder Halogen-Gruppen.

Vorzugsweise liegen maximal in einem der Strukturelemente -CR¹R² bzw. -CR³R⁴ Reste R¹ und R² bzw. R³ und R⁴ vor, die in einer Verbindung der allgemeinen Formel R¹-C(=O)-R² bzw. R³-C(=O)-R⁴ ein Riechstoffketon ergeben. Insbesondere liegen in beiden Strukturelementen -CR¹R² bzw. -CR³R⁴ Reste R¹ und R² bzw. R³ und R⁴ vor, die in einer Verbindung der allgemeinen Formel R¹-C(=O)-R² bzw. R³-C(=O)-R⁴ jeweils ein Riechstoffaldehyd ergeben, insbesondere das gleiche Riechstoffaldehyd.

R⁵ und R⁷ stehen, unabhängig voneinander, vorteilhafterweise jeweils für Wasserstoff oder einen C₁₋₆-Kohlenwasserstoffrest, der ggf. substituiert sein kann, vorzugsweise C₁₋₃- Kohlenwasserstoffrest. Besonders bevorzugt sind R⁵ und R⁷ jeweils Wasserstoff oder jeweils ein Methyl- oder Ethylrest, insbesondere aber jeweils Wasserstoff.

R¹ und R³ stehen, unabhängig voneinander, vorteilhafterweise jeweils für einen C₆₋₂₄-Kohlenwasserstoffrest, vorzugsweise C₇₋₂₄-Kohlenwasserstoffrest, wobei der Kohlenwasserstoffrest acyclisch oder cyclisch, substituiert oder unsubstituiert, verzweigt oder unverzweigt sowie gesättigt oder ungesättigt sein kann,

In einer weiter bevorzugten Ausführungsform bedeuten R², R⁴, R⁵, R⁷ Wasserstoff, R¹ und R³ bedeuten jeweils einen C₆₋₂₄-Kohlenwasserstoffrest, vorzugsweise C₇₋₂₄-Kohlenwasserstoffrest, wobei der Kohlenwasserstoffrest acyclisch oder cyclisch, substituiert oder unsubstituiert, verzweigt oder unverzweigt sowie gesättigt oder ungesättigt sein kann, und R⁶ steht für Wasserstoff oder einen C₁₋₂₄-Kohlenwasserstoffrest, welcher acyclisch oder cyclisch, substituiert oder unsubstituiert, verzweigt oder unverzweigt sowie gesättigt oder ungesättigt sein kann und welcher insbesondere mit ein oder zwei Hydroxylgruppen und/oder eine Aminogruppe substituiert sein kann, wobei auch bis zu 8 nicht benachbarte -CH₂-Gruppen durch -O- ersetzt sein können.

Bezogen auf R⁶ sind bevorzugte Reste C₁₋₁₆- Kohlenwasserstoffreste, insbesondere C₁₋₁₂- Kohlenwasserstoffreste, weiter bevorzugt C₁₋₆- Kohlenwasserstoffreste, am meisten bevorzugt C₁₋₃- Kohlenwasserstoffreste. Vorzugsweise handelt es sich um unverzweigte, acyclische Alkylreste. Sie können auch substituiert sein. Es kann sich z.B. um Mono- oder Dihydroxyalkylreste handeln, die anstelle der Hydroxylgruppen oder zusätzlich auch eine Aminogruppe aufweisen können. Sofern die Kohlenwasserstoffreste durch -O- unterbrochen sind, handelt es sich vorzugsweise um Strukturelemente der Formel -CH₂-CH₂-O- oder -CH₂-CH(CH₃)-O-. Derartige Verbindungen sind in einfacher Weise durch Alkoxylierung der entsprechenden Hydroxyverbindungen zugänglich. Ganz besonders bevorzugte Reste R⁶ sind Methyl-, Ethyl- oder Hydroxymethylreste oder Wasserstoff.

Demnach bedeuten in einer ganz besonders bevorzugten Ausführungsform die Reste R², R⁴, R⁵, R⁷ jeweils Wasserstoff, und der Rest R⁶ bedeutet einen Methyl-, Ethyl- oder Hydroxymethylrest oder Wasserstoff. Dabei bedeuten die Reste R¹ und R³ vorzugsweise jeweils einen C₆₋₂₄-Kohlenwasserstoffrest, insbesondere C₇₋₂₄-Kohlenwasserstoffrest, wobei der Kohlenwasserstoffrest acyclisch oder cyclisch, substituiert oder unsubstituiert, verzweigt oder unverzweigt sowie gesättigt oder ungesättigt sein.

Geeignete Oxazolidin-Riechstoffvorläufer gemäß der allgemeinen Formel (I) sind demnach z.B. 1-Aza-3,7-dioxa-2,8-diheptyl-bicyclo[3.3.0]octan, 1-Aza-3,7-dioxa-2,8-diheptyl-5-methyl-bicyclo[3.3.0]octan, 1-Aza-3,7-dioxa-2,8-diheptyl-5-hydroxymethyl-bicyclo[3.3.0]octan, 1-Aza-3,7-dioxa-2,8-diheptyl-5-ethyl-bicyclo[3.3.0]octan ,1-Aza-3,7-dioxa-2,8-dioctyl-bicyclo[3.3.0]octan, 1-Aza-3,7-dioxa-2,8-dioctyl-5-methyl-bicyclo[3.3.0]octan, 1-Aza-3,7-dioxa-2,8-dioctyl-5-hydroxymethyl-bicyclo[3.3.0]octan sowie 1-Aza-3,7-dioxa-2,8-dioctyl-5-ethyl-bicyclo[3.3.0]octan.

Die erfindungsgemäß einsetzbaren Verbindungen der allgemeinen Formel (I) sind insbesondere erhältlich durch Umsetzung von Verbindungen der allgemeinen Formel (II) 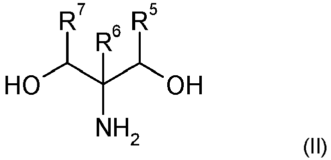 mit Verbindungen der allgemeinen Formeln R¹-C(=O)-R² und R³-C(=O)-R⁴ unter Ringschluss. Eine geeignete Substanz gemäß Formel (II) ist z.B. das 2-Aminopropan-1,3-diol. Für die Reste R¹ bis R⁶ gilt jeweils das zuvor bereits Geschriebene. Die allgemeinen Formeln R¹-C(=O)-R² und R³-C(=O)-R⁴ repräsentieren im Sinne der Erfindung ganz allgemeinen Riechstoff-Aldehyde bzw. Riechstoff-Ketone. Riechstoff-Aldehye sind diejenigen Riechstoffe, welche chemisch ein Aldehyd sind und welche vorteilhafterweise beim Menschen ein insbesondere angenehmes Geruchsempfinden auslösen. Riechstoff-Ketone sind diejenigen Riechstoffe, welche chemisch ein Keton sind und welche vorteilhafterweise beim Menschen ein insbesondere angenehmes Geruchsempfinden auslösen. Besonders geeignete Riechstoff-Aldehyde sowie Riechstoff-Ketone werden weiter unten exemplarisch aufgeführt. Zur Veranschaulichung seien zwei Beispiele gegeben. Beispielsweise steht bei dem Riechstoff-Aldehyd Octanal entsprechend der allgemeinen Formel R¹-C(=O)-R² der Rest R¹ für einen Heptylrest (also für CH₃-(CH₂)₆)- und der Rest R² steht für Wasserstoff oder umgekehrt. Beispielsweise steht bei dem Riechstoff-Keton Methylnonylketon entsprechend der allgemeinen Formel R¹-C(=O)-R² der Rest R¹ für einen Methylrest und der Rest R² steht für einen Nonylrest (also CH₃-(CH₂)₈-) oder umgekehrt.

Als Riechstoff-Aldehyde und/oder Riechstoff-Ketone können grundsätzlich alle üblichen Riechstoff-Aldehyde und/oder Riechstoff-Ketone eingesetzt werden, die insbesondere zur Herbeiführung eines angenehmen Geruchsempfindens beim Menschen eingesetzt werden. Solche Riechstoff-Aldehyde und/oder Riechstoff-Ketone sind dem Fachmann bekannt und auch in der Patentliteratur, beispielsweise in US 2003/0158079 A1, Absätze [0154] und [0155] beschrieben.

Besonders bevorzugte, im Sinne der Erfindung einsetzbare Riechstoff-Aldehyde sind Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-isopropylphenyl)butanal]), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)oxy]acetalde-hyd, 4-Isopropylbenzylaldehyd, 1,2,3,4,5,6,7,8-Octahydro-8,8-dmethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[ 5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimt-aldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexen-carboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro- 4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen- 1-al 3,5,5-Trimethylhexanal, Hexahydro-8,8-dimethyl-2-naphthaldehyd, 3-Propylbicyclo[ 2.2.1]-hept-5-en-2-carbaldehyd, 9-Decenal, 3-Methyl-5-phenyl-1-pentanal, Methylnonylacetaldehyd, Hexanal sowie trans-2-Hexenal. Die am meisten bevorzugten Riechstoff-Aldehyde im Sinne der Erfindung sind Lilial, Helional, Anisaldehyd, Cyclamenaldehyd, Triplal, Melonal, Methylundecanal, Undecanal, Nonanal sowie Octanal.

Besonders bevorzugte Riechstoff-Ketone im Sinne der Erfindung sind Methyl-beta-naphthylketon, Moschusindanon (1,2,3,5,6,7-hexahydro-1,1,2,3,3-pentamethyl-4H-inden-4-on), Tonalid (6-Acetyl-1,1,2,4,4,7-hexamethyltetralin ), alpha-Damascone, beta-Damascone, delta-Damascone, iso-Damascone, Damascenone, Methyldihydrojasmonat, Menthon, Carvon, Kampfer, Koavon (3,4,5,6,6-pentamethylhept-3-en-2-on), Fenchon, alpha-Ionon, beta-Ionon, gamma-Methyl-Ionon, Fleuramon (2-heptylcyclopentanon), Dihydrojasmon, cis-Jasmon, iso-E-Super (1-(1,2,3,4,5,6,7,8-octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3- methyl-5-propyl-2-cyclohexenon), 6-Isopropyldeca-hydro-2-naphton, Dimethyloctenon, Frescomenthe (2- butan-2-ylcyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopen-tanon, 1-(p-Menthen-6(2)yl)-1-propanon, 4-(4-Hydro-xy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl (4-(1,3-benzodioxol-5-yl) butan-2-on), Hexalon (1-(2,6,6-trimethyl-2-cyclohexene-1-yl)-1,6-heptadien-3-on), Isocyclemon E (2-acetonaphthon-1,2,3,4,5,6,7,8-octahydro-2,3,8,8-tetramethyl), Methylnonylketon, Methylcyclocitron, Methyllavendelketon, Orivon (4-tert-amylcyclohexanon), 4-tert- butyl cyclohexanon, Delphon (2-pentyl cyclopentanon), Muscon (CAS 541-91-3), Neobutenon (1-(5,5-dimethyl-1-cyclo-hexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on sowie Tetrameran (6,10-dimethylundecen-2-on) ist.

Für weitere geeignete Riechstoffe, ausgewählt aus Aldehyden und Ketonen, wird auf Steffen Arctander Published 1960 and 1969 respectively, Reprinted 2000 ISBN: Aroma Chemicals Vol. 1: 0-931710-37-5, Aroma Chemicals Vol. 2: 0-931710-38-3, verwiesen.

Wie aus den zuvor beispielhaft genannten Verbindungen erkennbar ist, können die Riechstoffaldehyde und -ketone eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen.

Zur Herstellung der erfindungsgemäß einzusetzenden Verbindungen der allgemeinen Formel (I) kann eine Verbindung der allgemeinen Formel (II) mit Aldehyden, Ketonen oder Mischungen von Ketonen und Aldehyden unter Ringschluß umgesetzt werden. Gemäß einer bevorzugten Ausführungsform der Erfindung leiten sich die Verbindungen der allgemeinen Formel (I) von einem Molekül der allgemeinen Formel (II) und zwei Aldehydmolekülen, welche gleich oder verschieden sein können, oder einem Aldehydmolekül und einem Ketonmolekül ab. Bei der Umsetzung von geringeren als stöchiometrischen Mengen an Aldehyden und/oder Ketonen liegen im Produktgemisch auch monocyclische Verbindungen vor. Der Anteil an bicyclischen Verbindungen zu monocyclischen Verbindungen kann aber in einfacher Weise durch Auswahl des Molverhältnisses zwischen Aldehyd/Keton und der Verbindung der allgemeinen Formel (II) eingestellt werden.

Die Umsetzung wird dabei vorzugsweise in einem geeigneten Lösungsmittel oder in situ durchgeführt. Geeignete Lösungsmittel sind beispielsweise aromatenhaltige Kohlenwasserstoffe wie Toluol. Die Umsetzung wird dabei vorzugsweise bei einer Temperatur im Bereich von 80 bis 150 °C, besonders bevorzugt 100 bis 140 °C durchgeführt. Beispielsweise wird die Verbindung der allgemeinen Formel (II) unter Stickstoffatmosphäre zusammen mit dem gewünschten Keton und/oder Aldehyd im Lösungsmittel vorgelegt. Sodann wird das Reaktionsgemisch erhitzt. Häufig wird sodann unter Rückfluss am Wasserabscheider erhitzt. Das erhaltene Umsetzungsprodukt wird nach üblichen Verfahren isoliert und gegebenenfalls gereinigt. In WO2007/087977 A1, auf welche hiermit Bezug genommen wird, wird die Herstellung von Verbindungen der allgemeinen Formel (I) auch anhand von Synthesebeispielen detailliert beschrieben.

Die Verbindungen der allgemeinen Formel (I) können unter Umgebungsbedingungen die gebundenen Riechstoffaldehyde und -ketone freisetzen. Umgebungsbedingungen sind dabei die typischen Umgebungsbedingungen im menschlichen Lebensraum bzw. die auf der menschlichen Haut anzutreffenden Bedingungen. Die Verbindungen der allgemeinen Formel (I) werden in den Parfümzusammensetzungen erfindungsgemäß als Mischungen mit den korrespondierenden Aldehyden oder Ketonen eingesetzt. Die erfindungsgemäßen Parfümzusammensetzungen zeichnen sich dadurch aus, dass das Molverhältnis von Riechstoff-Aldehyd und/oder Riechstoff-Keton zu dem korrespondierenden Oxazolidin-Riechstoffvorläufer 20:1 bis 1:20 vorzugsweise 10:1 bis 1:10, vorteilhafterweise 5:1 bis 1:5, weiter vorteilhaft 3:1 bis 1:3, noch vorteilhafter 2:1 bis 1:2 und insbesondere 1,2:1 bis 1:1,2 beträgt. Es konnte gefunden werden, dass solche Mischungen aus Riechstoff-Aldehyd und/oder Riechstoff-Keton und korrespondierenden Oxazolidin-Riechstoffvorläufer besonders gute Ergebnisse im Sinne dieser Erfindung hervorbringen, insbesondere mit Blick auf Stabilität und Wohlgeruch der Parfümzusammensetzung sowie der diese enthaltenden Prodkukte, sowie mit Blick auf den Duft der damit behandelten Objekte, wie insbesondere Textilien.

Am meisten bevorzugt sind dabei die entsprechenden Mischungen von Verbindungen der allgemeine Formel I, wobei die Reste R², R⁴, R⁵, R⁷ Wasserstoff bedeuten, die Reste R¹ und R³ jeweils einen C₆₋₂₄-Kohlenwasserstoffrest, (bevorzugt C₇₋₂₄-Kohlenwasser-stoffrest), wobei der Kohlenwasserstoffrest acyclisch oder cyclisch, substituiert oder unsubstituiert, verzweigt oder unverzweigt sowie gesättigt oder ungesättigt sein kann, bedeuten, und der Rest R⁶ für Wasserstoff oder einen Methyl-, Ethyl- oder Hydroxymethylrest steht, und wobei in beiden Strukturelementen -CR¹R² bzw. -CR³R⁴ die Reste R¹ und R² bzw. R³ und R⁴ in einer Verbindung der allgemeinen Formel R¹-C(=O)-R² bzw. R³-C(=O)-R⁴ jeweils einen Riechstoffaldehyd, vorzugsweise den gleichen Riechstoff-Aldehyd ergeben, insbesondere ausgewählt aus Lilial, Helional, Anisaldehyd, Cyclamenaldehyd, Triplal, Methylundecanal, Nonanal, Undecanal sowie Octanal.

Die erfindungsgemäße Parfümzusammensetzung kann im Sinne einer bevorzugten Ausführungsform der Erfindung im Wesentlichen nur aus den Komponenten (a) und (b) bestehen, also aus Riechstoff-Aldehyd und/oder Riechstoff-Keton sowie einem mit genau diesem Riechstoff-Aldehyd und/oder Riechstoff-Keton korrespondierenden Oxazolidin-Riechstoffvorläufer, welcher den gleichen Riechstoff-Aldehyd bzw. das gleiche Riechstoff-Keton freisetzen kann und welcher der allgemeinen Formel (I) genügt.

"Im Wesentlichen" bedeutet hier, dass diese bevorzugte Parfümzusammensetzung zu > 90 Gew.-%, vorzugsweise > 95 Gew.-% und insbesondere zu > 99 Gew.-% oder auch zu 100 Gew.-% aus den Komponenten (a) und (b) besteht. Die Parfümzusammensetzung kann grundsätzlich weitere Bestandteile umfassen, insbesondere weitere Riechstoffe und/oder Lösemittel umfassen. Ebenfalls können für Parfümzusammensetzungen typische Hilfsstoffe enthalten sein, wie z.B. Antioxidationsmittel (Sammelbezeichnung für Verbindungen verschiedenartiger chemischer Struktur, die unerwünschte, durch Sauerstoff-Einwirkung und andere oxidative Prozesse bedingte Veränderungen in den zu schützenden Zusammensetzungen hemmen oder verhindern), Konservierungsmittel (Sammelbezeichnung für Verbindungen verschiedenartiger chemischer Struktur, die unerwünschte, durch Einwirkung von Mikroorganismen oder Kleinlebewesen bedingte Veränderungen in den zu schützenden Zusammensetzungen hemmen oder verhindern) oder z.B. Fixateure.

Im Sinne einer weiteren bevorzugten Ausführungsform der Erfindung kann die erfindungsgemäße Parfümzusammensetzung aber auch größere Mengen weiterer Bestandteile, insbesondere weitere Riechstoffe und/oder Lösemittel, enthalten.

Fixateure, die als Hilfsstoffe optional einsetzbar sind, sind Stoffe, die Riechstoffen eine erhöhte Beständigkeit verleihen können. Als Fixateure sind insbesondere geeignet die sogenannten Eigenfixateure, die aufgrund ihrer Schwerflüchtigkeit ihren Eigengeruch lange bewahren, ohne dabei andere, leichter flüchtige Komponenten in ihrer Geruchsentfaltung zu behindern, wie insbesondere die synthetischen Moschuskörper, weiterhin die sogenannten Pseudofixateure als schwachriechende Stoffe, wie z.B. Diethylenglycolmethylether, sowie ferner die durch Adsorptionskräfte fixierenden Fixateure, wie insbesondere Extrakte aus Labdanum, Styrax, Tolubalsam, Benzoe, Iris, Eichenmoos oder Opopanax usw..

Geeignete optionale Lösungsmittel sind insbesondere die in der Parfümerie gebräuchlichen, wie vorzugsweise Dipropylengylcol, Diethylenglycol, Isopropylmyristat, Ethanol, Propylenglycol und/oder Rizinusöl. Andere geeignete optionale Hilfsstoffe sind z.B. Komplexbildner.

Gemäß einer besonders bevorzugten Ausführungsform der Erfindung enthält die erfindungsgemäße Parfümzusammensetzung weitere Riechstoffe.

Die Komponenten (a) und (b) können zusammen 100 Gew.-% der erfindungsgemäßen Parfümzusammensetzung ausmachen, was einer bevorzugten Ausführungsform entspricht. Wenn die Komponenten (a) und (b) in der erfindungsgemäße Parfümzusammensetzung in Summe in Mengen von 0,01-99 Gew.-%, vorzugsweise 0,1 bis 50 Gew.-%, insbesondere 1-30 Gew.-% enthalten sind, bezogen auf die gesamte Parfümzusammensetzung, so liegt eine weitere bevorzugte Ausführungsform der Erfindung vor. Geeignete Untergrenzen können dabei z.B. auch bei 2, 3, 4, 5, 10, 15, 20, 25 oder 30 Gew.-% liegen.

Gemäß einer bevorzugten Ausführungsform der Erfindung enthält die erfindungsgemäße Parfümzusammensetzung insgesamt > 20 Gew.-%, vorzugsweise > 30 Gew.-%, vorteilhafterweise > 40

Gew.-%, insbesondere > 50 Gew.-% Riechstoffe, wie z.B. > 60 Gew.-% oder > 70 Gew.-% Riechstoffe, bezogen auf die gesamte Zusammensetzung.

Grundsätzlich können die erfindungsgemäßen Parfümzusammensetzungen als optionale Hilfsstoffe auch Tenside enthalten. Es ist jedoch besonders bevorzugt, dass, sofern überhaupt Tenside enthalten sind, die erfindungsgemäße Parfümzusammensetzung < 15 Gew.-%, vorzugsweise < 5 Gew.-%, insbesondere < 1 Gew.-% Tenside umfasst. Der Tensidgehalt kann auch unter 10 Gew.-% oder unter 3 Gew.-% oder unter 0,5 Gew.-%, unter 0,1 Gew.-% oder unter 0,01 Gew.-% liegen. Wenn Tenside enthalten sind, was optional ist, dann kann eine geeignete Mindestmenge z.B. bei 0,0001 Gew.-% oder 0,001 Gew.-% liegen, Gew.-% jeweils bezogen auf die gesamte Zusammensetzung. Unter den Begriff der Tenside fallen im Sinne der Erfindung auch die Emulgatoren als grenzflächenaktive Stoffe. Bevorzugt einsetzbare Emulgatoren sind ethoxylierte Fettalkohole, ethoxylierte Triglyceride, Sorbitanfettsäureester, sowie hydriertes, ethoxyliertes Rizinusöl.

Gemäß einer weiteren bevorzugten Ausführungsform der Erfindung ist die erfindungsgemäße Parfümzusammensetzung flüssig oder gelartig. Sie kann grundsätzlich auch fest sein.

Die weiteren Riechstoffe, die in der erfindungsgemäßen Parfümzusammensetzung optional enthalten sein können, sind keinen besonderen Beschränkungen unterworfen. So können einzelne Riechstoffverbindungen natürlichen oder synthetischen Ursprungs, z.B. vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 - 18 C-Atomen, Citral, Citronellal, Citronellyloxyacetaldehyd, Cyclamenaldehyd (3-(4-propan-2-ylphenyl)butanal), Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen.

Die erfindungsgemäßen Parfümzusammensetzungen können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl. Weitere herkömmliche Riechstoffe, die im Rahmen der vorliegenden Erfindung in den erfindungsgemäßen Parfümzusammensetzungen enthalten sein können, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Do-decylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethyl-ether, Isosafrol, Jasmon, Kampfer, Karvakrol, Karvon, p-Kresolmethylether, Cumarin, p-Methoxy-acetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octyl-aldehyd, p-Oxy-Acetophenon, Pentadekanolid, β-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanilin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Die erfindungsgemäßen Parfümzusammensetzungen ermöglichen, wie bereits ausgeführt, Vorteile bei der Beduftung von Verbrauchsprodukten, wie insbesondere Wasch- oder Reinigungsmittel, da sie die Intensität des Produktgeruches als solches verbessern können und auch bei der Anwendung der Verbrauchsprodukte Duftvorteile bieten, insbesondere mit Blick auf einen lange anhaltenden Duft des behandelten Objektes. Die erfindungsgemäßen Parfümzusammensetzungen lassen sich problemlos und stabil in diverse Verbrauchsprodukte wie insbesondere Wasch- oder Reinigungsmittel, Kosmetika, Luftverbesserer, Klebstoffe einarbeiten. Besonders bevorzugte erfindungsgemäße Verbrauchsprodukte sind Wasch- oder Reinigungsmittel, wobei darunter im Sinne der Erfindung auch die Textilnachbehandlungsmittel wie insbesondere Weichspüler oder Hygienespüler zu subsumieren sind. Solche sind dem Fachmann an sich bekannt. Besonders bevorzugte Wasch- oder Reinigungsmittel sind feste, insbesondere pulverförmige Waschmittel, flüssige, insbesondere gelförmige Waschmittel sowie flüssige Weichspüler. Die Wasch- oder Reinigungsmittel können auch in Form von sogenannten Pouches (also in kleinen Beuteln), in Form von sogenannten Sheets (also Tüchern oder Folien) oder in Tablettenform vorliegen. Die erfindungsgemäßen Parfümzusammensetzungen können vor der Einarbeitung in das Wasch- oder Reinigungsmittel auch verkapselt werden.

Ein weiterer Gegenstand der Erfindung ist somit ein parfümiertes Verbrauchsprodukt (insbesondere Wasch- oder Reinigungsmittel, kosmetisches Mittel, Raumbeduftungsmittel und/oder Klebstoffe), welches eine erfindungsgemäße Parfümzusammensetzung wie zuvor beschrieben enthält. Das jeweilige Verbrauchsprodukt enthält weiterhin vorteilhafterweise die für das jeweilige Produkt üblichen Bestandteile. Besonders vorteilhaft ist der Einsatz der erfindungsgemäßen Parfümzusammensetzungen in Wasch- oder Reinigungsmitteln.

Ein weiterer Gegenstand der Erfindung ist ein Wasch- oder Reinigungsmittel, enthaltend eine erfindungsgemäße Parfümzusammensetzung in Mengen von 0,0001 bis 15 Gew.-%, Gew.-% bezogen auf das gesamte Mittel. Bevorzugte Untergrenzen für die erfindungsgemäße Parfümzusammensetzung in dem Wasch- oder Reinigungsmittel können auch bei 0,001 Gew.-%, 0,01 Gew.-% oder 0,1 Gew.-% liegen. Bevorzugte Obergrenzen für die erfindungsgemäße Parfümzusammensetzung in dem Wasch- oder Reinigungsmittel können auch bei 10 Gew.-%, 5 Gew.-% oder 3 Gew.-% liegen.

Als besonders vorteilhaft haben sich die erfindungsgemäßen Parfümzusammensetzungen und erfindungsgemäßen Wasch- oder Reinigungsmittel bei der Beduftung von Textilien erwiesen.

Dementsprechend ist ein weiterer Gegenstand der Erfindung ein Verfahren zur Beduftung von Textilien, wobei man die Textilien einem Textilbehandlungsprozess unter Einsatz einer erfindungsgemäße Parfümzusammensetzung oder eines erfindungsgemäßen Wasch- oder Reinigungsmittels unterwirft.

Bevorzugte Textilbehandlungsprozesse sind Waschprozesse, die manuell oder insbesondere maschinell, vorzugsweise in einer automatischen Waschmaschine durchgeführt werden können.

Als weiterer Vorteil hat sich herausgestellt, dass sogar nach maschineller Wäschetrocknung eine gute und auch lang anhaltende Textilbeduftung erzielt werden kann.

Weiterhin ist vorteilhaft, dass durch die Erfindung die Gesamtkonzentration an Parfüm im Produkt (z.B. im Waschmittel) reduzierbar ist, ohne einen Wohlgeruchsverlust zu erleiden. Es wird also eine effiziente Parfümierung ermöglicht.

Insgesamt wird eine Verlängerung der Duftwirkung des Wasch- oder -Reinigungsmittels ermöglicht. Weiterhin wird die Erzielung eines lange anhaltenden Frischegeruches bei der Anwendung des Wasch- oder Reinigungsmittels ermöglicht.

Ein weiterer Gegenstand der vorliegenden Erfindung liegt demnach in der Verwendung einer erfindungsgemäßen Parfümzusammensetzung in einem Wasch- oder Reinigungsmittel zur Verlängerung der Duftwirkung des Wasch- oder -Reinigungsmittels und/oder zur Erzielung eines lange anhaltenden Frischegeruches bei der Anwendung des Wasch- oder Reinigungsmittels.

Bevorzugt enthalten erfindungsgemäße Wasch oder Reinigungsmittel neben den erfindungsgemäßen Parfümzusammensetzungen wenigstens eine, vorzugsweise mehrere, aktive Komponenten, insbesondere wasch-, pflege- und/oder reinigungsaktive Komponenten, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen (Gerüststoffe), Bleichmittel, Bleichaktivatoren, Bleichstabilisatoren, Bleichkatalysatoren, Bügelhilfsmittel, Riechstoffe, Einlaufverhinderer, Elektrolyte, Enzyme, Farbschutzstoffe, Farbstoffe, Farbübertragungsinhibitoren, Fluoreszensmittel, Fungizide, Germizide, geruchskomplexierende Substanzen, Hydrotrope, Klarspüler, Komplexbildner, Konservierungsmittel, Korrosionsinhibitoren, optische Aufheller, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, UV-Schutz-Substanzen, Viskositätsregulatoren, Verdickungsmittel, Verfärbungsinhibitoren, Vergrauungsinhibitoren, Vitamine und/oder Avivage-Wirkstoffe.

Die Mengen der weiteren möglichen Inhaltsstoffe in den erfindungsgemäßen Wasch- oder Reinigungsmitteln orientieren sich jeweils am Einsatzzweck der betreffenden Mittel und der Fachmann ist mit den Größenordnungen der einzusetzenden Mengen der optionalen Inhaltsstoffe grundsätzlich vertraut oder kann diese der zugehörigen Fachliteratur entnehmen.

Je nach Einsatzzweck der erfindungsgemäßen Wasch- oder Reinigungsmittel wird man beispielsweise den Tensidgehalt höher oder niedriger wählen. Üblicherweise liegt z. B. der Tensidgehalt beispielsweise von Waschmitteln zwischen z.B. 5 und 50 Gew.-%, vorzugsweise zwischen 10 und 30 Gew.-% und insbesondere zwischen 15 und 25 Gew.-%, während Reinigungsmittel für das maschinelle Geschirrspülen üblicherweise zwischen z.B. 0,1 und 10 Gew.-%, vorzugsweise zwischen 0,5 und 7,5 Gew.-% und insbesondere zwischen 1 und 5 Gew.-% Tenside enthalten.

Die erfindungsgemäßen Wasch- oder Reinigungsmittel können vorzugsweise Tenside enthalten, wobei insbesondere anionische Tenside, nichtionische Tenside und deren Gemische, aber auch kationische Tenside in Frage kommen.

Zu den optional einsetzbaren nichtionischen Tensiden gehören die Alkoxylate, insbesondere die Ethoxylate und/oder Propoxylate, von gesättigten oder ein- bis mehrfach ungesättigten linearen oder verzweigtkettigen Alkoholen mit 10 bis 22 C-Atomen, vorzugsweise 12 bis 18 C-Atomen. Der Alkoxylierungsgrad der Alkohole liegt dabei in der Regel zwischen 1 und 20, vorzugsweise zwischen 3 und 10. Sie können in bekannter Weise durch Umsetzung der entsprechenden Alkohole mit den entsprechenden Alkylenoxiden hergestellt werden. Geeignet sind insbesondere die Derivate der Fettalkohole, obwohl auch deren verzweigtkettige Isomere, insbesondere sogenannte Oxoalkohole, zur Herstellung verwendbarer Alkoxylate eingesetzt werden können. Brauchbar sind demgemäß die Alkoxylate, insbesondere die Ethoxylate, primärer Alkohole mit linearen, insbesondere Dodecyl-, Tetradecyl-, Hexadecyl- oder Octadecyl-Resten sowie deren Gemische. Außerdem sind entsprechende Alkoxylierungsprodukte von Alkylaminen, vicinalen Diolen und Carbonsäureamiden, die hinsichtlich des Alkylteils den genannten Alkoholen entsprechen, verwendbar. Auch kommen die Ethylenoxid- und/oder Propylenoxid-Insertionsprodukte von Fettsäurealkylestern sowie Fettsäurepolyhydroxyamide, in Betracht.

Zur optionalen Einarbeitung in die erfindungsgemäßen Mittel geeignete sogenannte Alkylpolyglykoside sind Verbindungen der allgemeinen Formel (G)ₙ-OR⁸, in der R⁸ einen Alkyl- oder Alkenylrest mit 8 bis 22 C-Atomen, G eine Glykoseeinheit und n eine Zahl zwischen 1 und 10 bedeuten. Bei der Glykosidkomponente (G)ₙ handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad n nimmt als analytisch zu ermittelnde Größe im allgemeinen gebrochene Zahlenwerte an; er liegt bei Werten zwischen 1 und 10, bei den vorzugsweise eingesetzten Glykosiden unter einem Wert von 1,5, insbesondere zwischen 1,2 und 1,4. Bevorzugter Monomer-Baustein ist wegen der guten Verfügbarkeit Glucose. Der Alkyl- oder Alkenylteil R⁸ der Glykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Glykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders bevorzugte Alkylglykoside enthalten einen Kokosfettalkylrest, das heißt Mischungen mit im wesentlichen R⁸=Dodecyl und R⁸=Tetradecyl.

Nichtionisches Tensid ist in erfindungsgemäßen Wasch- oder Reinigungsmitteln vorzugsweise in Mengen von 0,1 Gew.-% bis 30 Gew.-%, insbesondere von 1 Gew.-% bis 25 Gew.-% optional enthalten, Gew.-% bezogen auf das gesamte Wasch- oder Reinigungsmittel.

Die Wasch- oder Reinigungsmittel können stattdessen oder zusätzlich weitere optionale Tenside enthalten, vorzugsweise Aniontenside.

Vorzugsweise sind Aniontenside des Sulfat- oder Sulfonat-Typs, in Mengen von vorzugsweise nicht über 30 Gew.-%, insbesondere von 0,1 Gew.-% bis 18 Gew.-%, jeweils bezogen auf gesamtes Wasch- oder Reinigungsmittel, optional enthalten. Als für den Einsatz in den erfindungsgemäßen Wasch- oder Reinigungsmittel besonders geeignete Aniontenside sind die Alkyl- und/oder Alkenylsulfate mit 8 bis 22 C-Atomen, die ein Alkali-, Ammonium- oder Alkyl- beziehungsweise Hydroxyalkyl-substituiertes Ammoniumion als Gegenkation tragen, zu nennen. Bevorzugt sind die Derivate der Fettalkohole mit insbesondere 12 bis 18 C-Atomen und deren verzweigtkettiger Analoga, der sogenannten Oxoalkohole. Die Alkyl- und Alkenylsulfate können in bekannter Weise durch Reaktion der entsprechenden Alkoholkomponente mit einem üblichen Sulfatierungsreagenz, insbesondere Schwefeltrioxid oder Chlorsulfonsäure, und anschließende Neutralisation mit Alkali-, Ammonium- oder Alkyl- beziehungsweise Hydroxyalkyl-substituierten Ammoniumbasen hergestellt werden. Derartige Alkyl- und/oder Alkenylsulfate sind in den Wasch- oder Reinigungsmitteln vorzugsweise in Mengen von 0,1 Gew.-% bis 20 Gew.-%, insbesondere von 0,5 Gew.-% bis 18 Gew.-% optional enthalten.

Zu den einsetzbaren Tensiden vom Sulfat-Typ gehören auch die sulfatierten Alkoxylierungsprodukte der genannten Alkohole, sogenannte Ethersulfate. Vorzugsweise enthalten derartige Ethersulfate 2 bis 30, insbesondere 4 bis 10, Ethylenglykol-Gruppen pro Molekül. Zu den einsetzbaren Aniontensiden vom Sulfonat-Typ gehören die durch Umsetzung von Fettsäureestern mit Schwefeltrioxid und anschließender Neutralisation erhältlichen α-Sulfoester, insbesondere die sich von Fettsäuren mit 8 bis 22 C-Atomen, vorzugsweise 12 bis 18 C-Atomen, und linearen Alkoholen mit 1 bis 6 C-Atomen, vorzugsweise 1 bis 4 C-Atomen, ableitenden Sulfonierungsprodukte, sowie die durch formale Verseifung aus diesen hervorgehenden Sulfofettsäuren.

Besonders bevorzugt optional einsetzbare Aniontenside sind die Alkylbenzolsulfonate, wie z.B. Natrium-Dodecylbenzolsulfonat.

Anionisches Tensid ist in erfindungsgemäßen Wasch- oder Reinigungsmitteln vorzugsweise in Mengen von 0,1 Gew.-% bis 30 Gew.-%, insbesondere von 1 Gew.-% bis 25 Gew.-% optional enthalten, Gew.-% bezogen auf das gesamte Wasch- oder Reinigungsmittel.

Als weitere fakultative tensidische Inhaltsstoffe der Wasch- oder Reinigungsmittel kommen Seifen in Betracht, wobei gesättigte Fettsäureseifen, wie die Salze der Laurinsäure, Myristinsäure, Palmitinsäure oder Stearinsäure, sowie aus natürlichen Fettsäuregemischen, zum Beispiel Kokos-, Palmkern- oder Talgfettsäuren, abgeleitete Seifen geeignet sind. Insbesondere sind solche Seifengemische bevorzugt, die zu 50 Gew.-% bis 100 Gew.-% aus gesättigten C₁₂-C₁₈-Fettsäureseifen und zu bis 50 Gew.-% aus Ölsäureseife zusammengesetzt sind. Vorzugsweise ist Seife in dem erfindungsgemäßen Wasch- oder Reinigungsmitteln in Mengen von 0,1 Gew.-% bis 5 Gew.-% optional enthalten. Insbesondere in flüssigen Wasch- oder Reinigungsmitteln können jedoch auch höhere Seifenmengen von bis zu 20 Gew.-% optional enthalten sein.

Auch Kationtenside können optional in den erfindungsgemäßen Wasch- oder Reinigungsmitteln enthalten sein. Beispiele für Kationtenside sind quartäre Ammonium-Verbindungen mit vorzugsweise einem oder insbesondere zwei hydrophoben Alkyl-Resten. Besonders bevorzugt sind Esterquats, also quartäre Ammonium-Verbindungen mit zwei hydrophoben Resten, die jeweils eine Ester-Gruppe als sogenannte Sollbruchstelle für einen leichteren biologischen Abbau enthalten. Bevorzugt einsetzbare Esterquats sind Methyl-N-(2-hydroxyethyl)-N,N-di(talgacyloxyethyl)ammonium-methosulfat, Bis-(pal-mitoyloxyethyl)-hydroxyethyl-methyl-ammonium-methosulfat, 1,2-Bis-[talgacyloxy]-3-trimethylammoniumpropanchlorid, N,N-Dimethyl-N,N-di(talgacyloxyethyl)ammonium-methosulfat oder Methyl-N,N-bis(stearoyloxyethyl)-N-(2-hydroxyethyl)ammonium-methosulfat.

Die kationischen Tenside sind in den erfindungsgemäßen Wasch- oder Reinigungsmitteln optional in Mengen von vorzugsweise 0,05 bis 20 Gew.%, bezogen auf das gesamte Wasch- oder Reinigungsmittel, enthalten. Mengen von 0,1 bis 5 Gew.% sind besonders bevorzugt.

Gemäß einer bevorzugten Ausführungsform der Erfindung sind Tenside in erfindungsgemäßen Wasch- oder Reinigungsmitteln in einer Gesamtmenge von vorzugsweise 5 Gew.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Wasch- oder Reinigungsmittel kann vorzugsweise mindestens einen Builder, vorzugsweise einen wasserlöslichen und/oder wasserunlöslichen, organischen und/oder anorganischen Builder enthalten. Bevorzugt ist der Einsatz wasserlöslicher Builder.

Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamin-tetrakis(methylenphosphonsäure) und 1-Hydroxyethan-1,1-diphosphonsäure, polymere Hydroxyverbin-dungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% in den erfindungsgemäßen Wasch- oder Reinigungsmittel enthalten sein. Mengen nahe der genannten Obergrenze werden vorzugsweise in pastenförmigen oder flüssigen, insbesondere wasserhaltigen, erfindungsgemäßen Wasch- oder Reinigungsmitteln eingesetzt. Erfindungsgemäße Wasch- oder Reinigungsmittel wie Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von z.B. bis zu 50 Gew.-%, vorzugsweise nicht über 40 Gew.-% und in flüssigen Mitteln insbesondere von 1 Gew.-% bis 5 Gew.-%, in den erfindungsgemäßen Wasch- oder Reinigungsmitteln optional eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln optional eingesetzt.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Wasch- oder Reinigungsmitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Amorphe Alkalisilikate sind bevorzugt.

Weiterhin ist es im Sinne einer weiteren Ausführungsform bevorzugt, allenfalls geringe Menge wasserunlöslicher Buildermaterialien (wie z.B. Zeolith) einzusetzen, beispielsweise in Mengen von 0-5 Gew.-%, z.B. 0,1 bis 2 Gew.-%, bezogen auf das gesamte Wasch- oder Reinigungsmittel.

Buildersubstanzen sind in den erfindungsgemäßen Wasch- oder Reinigungsmitteln vorzugsweise in Mengen bis zu 60 Gew.-%, insbesondere von 5 Gew.-% bis 40 Gew.-%, optional enthalten. Erfindungsgemäße Wäschenachbehandlungsmittel, wie z.B. Weichspüler, sind vorzugsweise frei von anorganischem Builder.

Als optional einsetzbare Persauerstoffverbindungen kommen insbesondere organische Persäuren beziehungsweise persaure Salze organischer Säuren, wie Phthalimidpercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt kann Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt werden. Falls ein erfindungsgemäßes Wasch- oder Reinigungsmittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der optionale Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, optional eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacet-oxy-2,5-dihydrofuran und Enolester sowie acetyliertes Sorbitol und Mannitol beziehungsweise deren Mischungen , acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, z.B. N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, optional enthalten sein.

Als in den Wasch- oder Reinigungsmitteln optional einsetzbare Enzyme kommen insbesondere solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- oder Reinigungsmitteln, vorzugsweise in Mengen nicht über 5 Gew.-%, insbesondere von 0,2 Gew.-% bis 2 Gew.-%, optional enthalten.

Die Wasch- oder Reinigungsmittel können als optische Aufheller beispielsweise Derivate der Diaminostilbendisulfonsäure beziehungsweise deren Alkalimetallsalze optional enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den optional einsetzbaren Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen können auch Gemische aus verschiedenen Schauminhibitoren verwendet werden, zum Beispiel solche aus Silikonen, Paraffinen oder Wachsen. Vorzugsweise sind die optionalen Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Wasch- oder Reinigungsmittel optional auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Waschmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Wasch- oder Reinigungsmittel können optional auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone, N-Vinyl-imidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren.

Die optional einsetzbaren Vergrauungsinhibitoren haben das Vermögen, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf das Wasch- oder Reinigungsmittel, als optionale Vergrauungsinhibitoren eingesetzt werden.

Zu den in den erfindungsgemäßen Wasch- oder Reinigungsmitteln, insbesondere wenn sie in flüssiger oder pastöser Form vorliegen, optional verwendbaren organischen Lösungsmitteln gehören vorzugsweise Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel können in den erfindungsgemäßen Wasch- oder Reinigungsmitteln vorzugsweise in Mengen von nicht über 30 Gew.-%, insbesondere von 6 Gew.-% bis 20 Gew.-%, optional vorhanden sein.

Zur Einstellung eines gewünschten, sich durch die Mischung der übrigen Komponenten nicht von selbst ergebenden pH-Werts können die erfindungsgemäßen Wasch- oder Reinigungsmittel Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, optional enthalten. Derartige pH-Regulatoren können in den erfindungsgemäßen Wasch- oder Reinigungsmitteln vorzugsweise nicht über 20 Gew.-%, insbesondere von 1,2 Gew.-% bis 17 Gew.-%, optional enthalten sein.

Die Herstellung fester erfindungsgemäßer Wasch- oder Reinigungsmittel kann in im Prinzip bekannter Weise, zum Beispiel durch Sprühtrocknen oder Granulation, erfolgen, wobei z.B. optionale Persauerstoffverbindung und optionaler Bleichkatalysator gegebenenfalls später zugesetzt werden. Die erfindungsgemäße Parfümzusammensetzung wird vorzugsweise zum Ende der Herstellung in das Wasch- oder Reinigungsmittel eingebracht, vorzugsweise durch Aufsprühen. Zur Herstellung erfindungsgemäßer Wasch- oder Reinigungsmittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Wasch- oder Reinigungsmittel kann ebenfalls in an sich bekannter Weise erfolgen, wobei die erfindungsgemäße Parfümzusammensetzung vorzugsweise zum Ende der Herstellung in das Wasch- oder Reinigungsmittel eingebracht wird.

Gemäß einer bevorzugten Ausführungsform kann die erfindungsgemäße Lehre dazu eingesetzt werden, den Parfümanteil in Verbrauchsprodukten wie Wasch-, Reinigungs- und Körperpflegemitteln herabzusetzen, denn durch die Inkorporation der erfindungsgemäßen Parfümzusammensetzung kann nicht nur eine besonders lang anhaltende, sondern auch eine besonders effiziente Parfümierung gewährleistet werden.

Ein bevorzugtes erfindungsgemäßes Wasch- oder Reinigungsmittel ist ein festes, insbesondere pulverförmiges Waschmittel, das neben der erfindungsgemäßen Parfümzusammensetzung vorzugsweise Komponenten enthalten kann, die vorzugsweise ausgewählt sind aus den folgenden:
(a) Aniontenside, wie vorzugsweise Alkylbenzolsulfonat, Alkylsulfat, z.B. in Mengen von vorzugsweise 5-30 Gew.-%
(b) Nichtionische Tenside, wie vorzugsweise Fettalkoholpolyglycolether, Alkylpolyglucosid, Fettsäureglucamid z.B. in Mengen von vorzugsweise 0,5-15 Gew.-%
(c) Gerüststoffe, wie z.B. Polycarboxylat, Natriumcitrat, in Mengen von z.B. 0-70 Gew.-%, vorteilhafterweise 5-60 Gew.-%, vorzugsweise 10-55 Gew.-%, insbesondere 15-40 Gew.-%,
(d) Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
(e) Bleichmittel, wie z.B. Natriumperborat oder Natriumpercarbonat, in Mengen von z.B. 0-30 Gew.-% vorteilhafterweise 5-25 Gew.-%, vorzugsweise 10-20 Gew.-%,
(f) Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
(g) Stabilisatoren, z.B. Phosphonate, vorteilhafterweise 0-1 Gew.-%,
(h) Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
(i) Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
(j) Vergrauungsinhibitor, z.B. Carboxymethylcellulose, vorteilhafterweise 0-1 Gew.-%,
(k) Verfärbungsinhibitor, z.B. Polyvinylpyrrolidon-Derivate, z.B. 0-2 Gew.-%,
(l) Stellmittel, z.B. Natriumsulfat, vorteilhafterweise 0-20 Gew.-%,
(m) Optische Aufheller, z.B. Stilben-Derivat, Biphenyl-Derivat, vorteilhafterweise 0-0,4 Gew.-%, insbesondere 0,1-0,3 Gew.-%,
(n) ggf. weitere Riechstoffe
(o) ggf. Wasser
(p) ggf. Seife
(q) ggf. Bleichaktivatoren
(r) ggf. Cellulosderivate
(s) ggf. Schmutzabweiser,

Gew.-% jeweils bezogen auf das gesamte Mittel.

In einer weiter bevorzugten Ausführungsform ist das erfindungsgemäße Wasch- oder Reinigungsmittel festförmig, insbesondere teilchenförmig und enthält neben der erfindungsgemäßen Parfümzusammen-setzung noch 5 Gew.-% bis 55 Gew.-% Gerüststoffe, 2,5 Gew.-% bis 20 Gew.-% Aniontensid, 1 Gew.-% bis 20 Gew.-% Niotensid, 1 Gew.-% bis 25 Gew.-% Bleichmittel, 0,5 Gew.-% bis 8 Gew.-% Bleich-aktivator und 0,1 Gew.-% bis 40 Gew.-% Stellmittel, insbesondere Alkalisulfat, sowie bis zu 2 Gew.-%, insbesondere 0,4 Gew.-% bis 1,2 Gew.-% Enzym, vorzugsweise teilchenförmig konfektioniertes Enzym, insbesondere Protease, Lipase, Amylase, Cellulase und/oder Oxidoreduktase. Diese Ausführungsform kann optional auch frei von Bleichmittel und Bleichaktivator sein.

In einer anderen bevorzugten Ausführungsform der Erfindung liegt das erfindungsgemäße Wasch- oder Reinigungsmittel in flüssiger Form vor, vorzugsweise in Gelform. Bevorzugte flüssige Wasch- oder Reinigungsmittel haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. ≤ 30 Gew.-%, vorzugsweise ≤ 20 Gew.-%, insbesondere ≤ 15 Gew.-%, wie z.B. 0,1 bis 10 Gew.-%, betragen, Gew.-% jeweils bezogen auf das gesamte Mittel. Die flüssigen Verbrauchsprodukte können auch nichtwässrige Lösungsmittel enthalten.

Ein bevorzugtes erfindungsgemäßes Wasch- oder Reinigungsmittel ist ein flüssiges, insbesondere gelförmiges Waschmittel, das neben den erfindungsgemäßen Parfümzusammensetzungen vorzugsweise Komponenten enthalten kann, die vorzugsweise ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Polycarboxylat, Natriumcitrat, vorteilhafterweise 0-25 Gew.-%, vorzugsweise 0,01-10 Gew.-%, insbesondere 0,1-5 Gew.-%,
- Schauminhibitor, z.B. Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Riechstoffe
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein besonders bevorzugtes flüssiges Wasch- oder Reinigungsmittel enthält dabei neben den erfindungsgemäßen Parfümzusammensetzungen zumindest Aniontenside in Mengen von 0,5 Gew.-% bis 20 Gew.-%, nichtionische Tenside in Mengen von 1 Gew.-% bis 25 Gew.-%, Gerüststoffe in Mengen von 1 bis 25 Gew.-%, Enzyme sowie Wasser.

Ein weiteres bevorzugtes erfindungsgemäßes Wasch- oder Reinigungsmittel ist ein flüssiger Weichspüler, der neben den erfindungsgemäßen Parfümzusammensetzungen vorzugsweise Komponenten enthalten kann, die ausgewählt sind aus den folgenden:
- Kationische Tenside, wie insbesondere Esterquats, z.B. in Mengen von 5-30 Gew.-%,
- Cotenside, wie insbesondere Glycerolmonostearat, Stearinsäure, Fettalkohole und/oder Fettalkoholethoxylate, z.B. in Mengen von 0-5 Gew.-%, vorzugsweise 0,1-4 Gew.-%,
- Emulgatoren, wie insbesondere Fettaminethoxylate, z.B. in Mengen von 0-4 Gew.-%, vorzugsweise 0,1-3 Gew.-%,
- ggf. weitere Riechstoffe
- ggf. Farbstoffe, vorzugsweise im ppm-Bereich
- Lösemittel, wie insbesondere Wasser, z.B. in Mengen von 60-90 Gew.-%,

Gew.-% jeweils bezogen auf das gesamte Mittel.

### Beispiele:

In einer Waschmaschine vom Typ Miele® Softtronic W1734 wurden im Rahmen von Waschversuchen bei 40 °C Frotteehandtücher (Gesamtgewicht je 3,5 kg) aus Baumwolle gewaschen (Wasserhärte 16 °d), geschleudert (Schleuderdrehzahl 1200 U/min) und danach an der Leine getrocknet. Die Waschversuche wurden unabhängig voneinander (a) mit jeweils 40 g eines bleichmittelhaltigen festen Universalwaschmittels vom Typ Megaperls® (hochverdichtetes festes Waschmittel erhältlich über Extrusionsverfahren) sowie (b) mit jeweils 75 ml eines flüssigen Universalwaschmittels gewaschen.

Das bleichmittelhaltige feste Universalwaschmittel enthielt insgesamt 0,4 Gew.-% Parfümöl. Das Parfümöl enthielt dabei in Variante a1) 10 Gew.-% Octanal, in Variante a2) 10 Gew.-% einer Mischung aus Octanal und einem korrespondierenden Oxazolidin (1-Aza-3,7-dioxa-2,8-diheptyl-bicyclo[3.3.0]oc-tan), in Variante a3) 10 Gew.-% 1-Aza-3,7-dioxa-2,8-diheptyl-bicyclo[3.3.0]octan, Gew.-% jeweils bezogen auf das Parfümöl. Die konkrete Octanal-Oxazolidin-Mischung gemäß Variante a2) umfasste 30 Gew.-% Octanal und 70 Gew.-% Oxazolidin, bezogen auf die Octanal-Oxazolidin-Mischung.

Das flüssige Universalwaschmittels enthielt insgesamt 1,3 Gew.-% Parfümöl. Das Parfümöl enthielt dabei in Variante b1) 10 Gew.-% Octanal, in Variante b2) 10 Gew.-% einer Mischung aus Octanal und einem korrespondierenden Oxazolidin (1-Aza-3,7-dioxa-2,8-diheptyl-bicyclo[3.3.0]octan), in Variante b3) 10 Gew.-% 1-Aza-3,7-dioxa-2,8-diheptyl-bicyclo[3.3.0]octan, Gew.-% jeweils bezogen auf das Parfümöl. Die konkrete Octanal-Oxazolidin-Mischung gemäß Variante b2) umfasste 30 Gew.-% Octanal und 70 Gew.-% Oxazolidin, bezogen auf die Octanal-Oxazolidin-Mischung

Geprüft wurden jeweils der Produktgeruch (also der Geruch des Waschmittels), der Geruch der trockenen Wäsche (d.h. 1 Tag nach der Wäsche) sowie der Geruch der trockenen Wäsche nach 7 Tagen (d.h. 7 Tage nach der Wäsche). Zur Lagerung wurden die Handtücher zusammengelegt und in einem offenen Regal jeweils gestapelt.

Bewertet wurde dabei jeweils die Intensität des Geruches auf einer Skala von 1 bis 5, wobei der Wert 5 der Maximalwert ist und einen sehr intensiven Geruch beschreibt und der Wert 1 der Minimalwert ist, der einen noch wahrnehmbaren Geruch beschreibt. Die Bewertung wurde von 10 parfümistisch geschulten Personen durchgeführt. Jede Bewertung wurde jeweils viermal wiederholt und dann der Durchschnittswert bestimmt.

### (a) Auswertung festes Universalwaschmittel

Der Produktgeruch des Mittels gemäß Variante a1) (Parfümöl enthaltend Octanal) wurde mit dem Durchschnittswert 3,5 bewertet. Der Produktgeruch des Mittels gemäß Variante a2) (Parfümöl enthaltend Octanal und korrespondierendes Oxazolidin) wurde mit dem Durchschnittswert 4,5 bewertet. Der Produktgeruch des Mittels gemäß Variante a3) (Parfümöl enthaltend Oxazolidin) wurde mit dem Durchschnittswert 3 bewertet.

Die trockene Wäsche, gewaschen mit Mittel gemäß Variante a1), wurde mit dem Durchschnittswert 3 bewertet. Die trockene Wäsche, gewaschen mit Mittel gemäß Variante a2), wurde mit dem Durchschnittswert 5 bewertet. Die trockene Wäsche, gewaschen mit Mittel gemäß Variante a3), wurde mit dem Durchschnittswert 4,5 bewertet.

Die trockene Wäsche, gewaschen mit Mittel gemäß Variante a1), wurde nach 7 Tagen Lagerung mit dem Durchschnittswert 2 bewertet. Die trockene Wäsche, gewaschen mit Mittel gemäß Variante a2), wurde nach 7 Tagen Lagerung mit dem Durchschnittswert 5 bewertet. Die trockene Wäsche, gewaschen mit Mittel gemäß Variante a3), wurde nach 7 Tagen Lagerung mit dem Durchschnittswert 4,5 bewertet.

Demnach fand man beim Einsatz der erfindungsgemäßen Mischung im festen Universalwaschmittel, entsprechend Variante a2), sowohl Wohlgeruchsvorteile bei dem Waschmittel als solchem wie auch bei der trockenen Wäsche und 7 Tage gelagerter trockener Wäsche.

### (b) Auswertung flüssiges Universalwaschmittel

Der Produktgeruch des Mittels gemäß Variante b1) (Parfümöl enthaltend Octanal) wurde mit dem Durchschnittswert 4 bewertet. Der Produktgeruch des Mittels gemäß Variante b2) (Parfümöl enthaltend Octanal und korrespondierendes Oxazolidin) wurde mit dem Durchschnittswert 4,5 bewertet. Der Produktgeruch des Mittels gemäß Variante b3) (Parfümöl enthaltend Oxazolidin) wurde mit dem Durchschnittswert 3,5 bewertet.

Die trockene Wäsche, gewaschen mit Mittel gemäß Variante b1), wurde mit dem Durchschnittswert 3 bewertet. Die trockene Wäsche, gewaschen mit Mittel gemäß Variante b2), wurde mit dem Durchschnittswert 4 bewertet. Die trockene Wäsche, gewaschen mit Mittel gemäß Variante b3), wurde mit dem Durchschnittswert 3 bewertet.

Die trockene Wäsche, gewaschen mit Mittel gemäß Variante b1), wurde nach 7 Tagen Lagerung mit dem Durchschnittswert 1,5 bewertet. Die trockene Wäsche, gewaschen mit Mittel gemäß Variante b2), wurde nach 7 Tagen Lagerung mit dem Durchschnittswert 3,5 bewertet. Die trockene Wäsche, gewaschen mit Mittel gemäß Variante b3), wurde nach 7 Tagen Lagerung mit dem Durchschnittswert 3 bewertet.

Demnach fand man beim Einsatz der erfindungsgemäßen Mischung im flüssigen Universalwaschmittel gemäß Variante b2) sowohl Wohlgeruchsvorteile bei dem Waschmittel als solchen wie auch bei der trockenen Wäsche und 7 Tage gelagerter trockener Wäsche.

Analoge Waschversuche unter Einsatz entsprechender Softener ergaben vergleichbare Ergebnisse, welche die Vorteilhaftigkeit der erfindungsgemäßen Mischungen für Produktgeruch und Duft der trockenen Wäsche zeigten.

## Patentansprüche

1. Parfümzusammensetzung, umfassend
(a) zumindest einen Riechstoff-Aldehyd mit mindestens 6 Kohlenstoffatomen oder ein Riechstoff-Keton mit mindestens 6 Kohlenstoffatomen
sowie
(b) zumindest einen mit dem unter (a) genannten Riechstoff-Aldehyd oder Riechstoff-Keton korrespondierenden Oxazolidin-Riechstoffvorläufer, welcher den gleichen Riechstoff-Aldehyd bzw. das gleiche Riechstoff-Keton freisetzen kann,
wobei das Molverhältnis von Riechstoff-Aldehyd und/oder Riechstoff-Keton (a) zu dem korrespondierenden Oxazolidin-Riechstoffvorläufer (b) 20:1 bis 1:20, vorzugsweise 10:1 bis 1:10, vorteilhafterweise 5:1 bis 1:5 beträgt und
wobei es sich bei dem Oxazolidin-Riechstoffvorläufer um eine 1-Aza-3,7-dioxabicyclo[3.3.0]octan-Verbindung der allgemeinen Formel (I) handelt 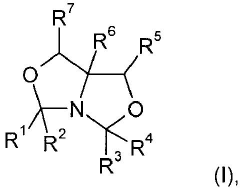 wobei
R¹, R², R³, R⁴ unabhängig voneinander für Reste stehen, die in einer Verbindung der allgemeinen Formel R¹-C(=O)-R² bzw. R³-C(=O)-R⁴ einen Riechstoff-Aldehyd mit mindestens 6 Kohlenstoffatomen oder ein Riechstoff-Keton mit mindestens 6 Kohlenstoffatomen ergeben,
R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann.

2. Zusammensetzung gemäß Anspruch 1, **dadurch gekennzeichnet, dass** der Riechstoff-Aldehyd ausgewählt ist aus Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-isopropylphenyl)-butanal]), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), 4-Methoxybenzaldehyd, Benzaldehyd, 3-(4-tert-Butylphenyl)-propanal, 2-Methyl-3-(para-methoxyphenyl)-propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dmethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[ 5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydro-zimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carbox-aldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyl-octan-1-al, 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolace-taldehyd, 6,6-Dimethyl-2-norpinen-2-propionaldehyd, para-Methylphenoxyacetaldehyd, 2-Methyl-3-phenyl-2-propen-1-al 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

3. Zusammensetzung gemäß Anspruch 1 oder 2, **dadurch gekennzeichnet, dass** das Riechstoff-Keton ausgewählt ist aus Methyl-beta-naphthylketon, Moschusindanon (1,2,3,5,6,7-hexahydro-1,1,2,3,3-pentamethyl-4H-inden-4-on), Tonalid (6-Acetyl-1,1,2,4,4,7-hexamethyltetralin ), alpha-Damascone, beta-Damascone, delta-Damascone, iso-Damascone, Damascenone, Methyldihydrojasmonat, Menthon, Carvon, Kampfer, Koavon (3,4,5,6,6-pentamethylhept-3-en-2-on), Fenchon, alpha-Ionon, beta-Ionon, gamma-Methyl-Ionon, Fleuramon (2-heptylcyclopentanon), Dihydrojasmon, cis-Jasmon, iso-E-Super (1-(1,2,3,4,5,6,7,8-octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3- methyl-5-propyl-2-cyclohexenon), 6-Isopropyldecahydro-2-naphton, Dimethyloctenon, Frescomenthe (2- butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)-cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-benzodioxol-5-yl) butan-2-on), Hexalon (1-(2,6,6-trimethyl-2-cyclohexene-1-yl)-1,6-heptadien-3-on), Isocyclemon E (2-acetonaphthon-1,2,3,4,5,6,7,8-octahydro-2,3,8,8-tetramethyl), Methylnonylketon, Methylcyclocitron, Methyllavendelketon, Orivon (4-tert-amyl-cyclohexanon), 4-tert- butyl cyclohexanon, Delphon (2-pentyl cyclopentanon), Muscon (CAS 541-91-3), Neobutenon (1-(5,5-dimethyl-1-cyclohexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on sowie Tetrameran (6,10-dimethylundecen-2-on).

4. Zusammensetzung nach einem der Ansprüche 1 bis 3, **dadurch gekennzeichnet, dass** die Reste R², R⁴, R⁵, R⁷ jeweils für Wasserstoff stehen, und der Rest R⁶ für einen Methyl-, Ethyl- oder Hydroxymethylrest oder Wasserstoff steht, sowie dass die Reste R¹ und R³ unabhängig voneinander, jeweils für einen C₆₋₂₄-Kohlenwasserstoffrest, vorzugsweise C₇₋₂₄-Kohlenwasserstoffrest stehen, wobei der Kohlenwasserstoffrest acyclisch oder cyclisch, substituiert oder unsubstituiert, verzweigt oder unverzweigt sowie gesättigt oder ungesättigt sein kann.

5. Zusammensetzung nach einem der Ansprüche 1 bis 4, **dadurch gekennzeichnet, dass** diese weitere Riechstoffe enthält.

6. Zusammensetzung nach einem Ansprüche 1 bis 5, **dadurch gekennzeichnet, dass** die Komponenten (a) und (b) in Mengen von 0,1 bis 99 Gew.-%, vorzugsweise 1 bis 50 Gew.-%, insbesondere 5 bis 30 Gew.-% enthalten sind, bezogen auf die gesamte Zusammensetzung.

7. Parfümiertes Verbrauchsprodukt, insbesondere Wasch- oder Reinigungsmittel, kosmetisches Mittel, Raumbeduftungsmittel oder Klebstoffe, **dadurch gekennzeichnet**, das es eine Zusammensetzung nach einem der Ansprüche 1 bis 6 enthält.

8. Wasch- oder Reinigungsmittel enthaltend eine Zusammensetzung nach einem der Ansprüche 1 bis 6 in Mengen von 0,0001 bis 15 Gew.-%, bezogen auf das gesamte Mittel.

9. Verfahren zur Beduftung von Textilien, **dadurch gekennzeichnet, dass** man die Textilien einem Waschprozess unter Einsatz einer Zusammensetzung nach einem der Ansprüche 1 bis 6 oder eines Wasch- oder Reinigungsmittels nach Anspruch 8 unterwirft.

10. Verwendung einer Parfümzusammensetzung nach einem der Ansprüche 1 bis 6 in einem Wasch- oder Reinigungsmittel zur Verlängerung der Duftwirkung des Wasch- oder Reinigungsmittels und/oder zur Erzielung eines lange anhaltenden Frischegeruches bei der Anwendung des Wasch- oder Reinigungsmittels.

## Claims

1. A perfume composition comprising
(a) at least one odorant aldehyde having at least 6 carbon atoms or one odorant ketone having at least 6 carbon atoms
and
(b) at least one oxazolidine odorant precursor, corresponding with the odorant aldehyde or odorant ketone named in (a), which can release the same odorant aldehyde or the same odorant ketone, respectively,
wherein the molar ratio of odorant aldehyde and/or odorant ketone (a) to the corresponding oxazolidine odorant precursor (b) is from 20:1 to 1:20, preferably from 10:1 to 1:10, advantageously 5:1 to 1:5, and
wherein the oxazolidine odorant precursor is a 1-aza-3,7-dioxabicyclo[3.3.0]octane compound of general formula (I), 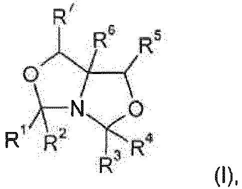 in which
R¹, R², R³ and R⁴, independently of one another, represent functional groups which produce an odorant aldehyde having at least 6 carbon atoms or an odorant ketone having at least 6 carbon atoms in a compound of the general formula R¹-C(=O)-R² or R³-C(=O)-R⁴, respectively,
R⁵, R⁶ and R⁷, independently of one another, represent H or a hydrocarbon functional group which can be acyclic or cyclic, substituted or unsubstituted, branched or unbranched, and saturated or unsaturated.

2. The composition according to claim 1, **characterized in that** the odorant aldehyde is selected from Adoxal (2,6,10-trimethyl-9-undecenal), anisaldehyde (4-methoxybenzaldehyde), cymene (3-(4-isopropylphenyl)-2-methylpropanal), ethylvanillin, Florhydral (3-(3-isopropylphenyl)-butanal]), helional (3-(3,4-methylenedioxyphenyl)-2-methylpropanal), heliotropin, hydroxycitronellal, lauraldehyde, Lyral (3- and 4-(4-hydroxy-4-methylpentyl)-3-cyclohexene-1-carboxaldehyde), methylnonylacetaldehyde, Lilial (3-(4-tert-butylphenyl)-2-methylpropanal), phenylacetaldehyde, undecylenic aldehyde, vanillin, 2,6,10-trimethyl-9-undecenal, 3-dodecene-1-al, alpha-n-amylcinnamaldehyde, Melonal (2,6-dimethyl-5-heptenal), 2,4-dimethyl-3-cyclohexene-1-carboxaldehyde (Triplal), 4-methoxybenzaldehyde, benzaldehyde, 3-(4-tert-butylphenyl)-propanal, 2-methyl-3-(para-methoxyphenyl)-propanal, 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzylaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, 2,6-dimethyl-5-heptenal, 4-(tricyclo[5.2.1.0(2,6)]-decylidene-8)-butanal, octahydro-4,7-methano-1H-indenecarboxaldehyde, 3-ethoxy-4-hydroxybenzaldehyde, para-ethyl-alpha,alpha-dimethyl hydrocinnamaldehyde, alpha-methyl-3,4-(methylenedioxy)-hydrocinnamaldehyde, 3,4-methylenedioxybenzaldehyde, alpha-n-hexylcinnamaldehyde, m-cymene-7-carboxaldehyde, alpha-methylphenylacetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3-cyclohexenecarboxaldehyde, 1-dodecanal, 2,4-dimethylcyclohexene-3-carboxaldehyde, 4-(4-hydroxy-4-methylpentyl)-3-cyclohexene-1-carboxaldehyde, 7-methoxy-3,7-dimethyl-octan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanoindane-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexenecarboxaldehyde, 7-hydroxy-3,7-dimethyloctanal, trans-4-decenal, 2,6-nonadienal, para-tolylacetaldehyde, 4-methylphenylacetaldehyde, 2-methyl-4-(2,6,6-trimethyl-1-cyclohexen-1-yl)-2-butenal, ortho-methoxycinnamaldehyde, 3,5,6-trimethyl-3-cyclohexenecarboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindane-1-carboxaldehyde, 2-methyloctanal, alpha-methyl-4-(1-methylethyl)benzolacetaldehyde, 6,6-dimethyl-2-norpinene-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methylnonyl acetaldehyde, hexanal, and trans-2-hexenal.

3. The composition according to claim 1 or claim 2, **characterized in that** the odorant ketone is selected from methyl-beta-naphthylketone, musk indanone (1,2,3,5,6,7-hexahydro-1,1,2,3,3-pentamethyl-4H-inden-4-one), tonalide (6-acetyl-1,1,2,4,4,7-hexamethyltetralin), alpha-damascone, beta-damascone, delta-damascone, iso-damascone, damascenone, methyl dihydrojasmonate, menthone, carvone, camphor, Koavone (3,4,5,6,6-pentamethylhept-3-en-2-one), fenchone, alpha-ionone, beta-ionone, gamma-methyl ionone, Fleuramone (2-heptylcyclopentanone), dihydrojasmone, cis-jasmone, Iso E Super (1-(1,2,3,4,5,6,7,8-octahydro-2,3,8,8-tetramethyl-2-naphthalenyl)-ethan-1-one (and isomers)), methyl cedrenyl ketone, acetophenone, methylacetophenone, para-methoxyacetophenone, methyl-beta-naphtylketone, benzylacetone, benzophenone, para-hydroxy phenyl butanone, celery ketone (3-methyl-5-propyl-2-cyclohexenone), 6-isopropyldecahydro-2-naphtone, dimethyloctenone, Frescomenthe (2-butan-2-yl-cyclohexan-1-one), 4-(1-ethoxyvinyl)-3,3,5,5-tetramethylcyclohexanone, methylheptenone, 2-(2-(4-methyl-3-cyclohexen-1-yl)propyl)-cyclopentanone, 1-(p-menthen-6(2)yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)-indanone, 4-damascol, Dulcinyl (4-(1,3-benzodioxol-5-yl) butan-2-one), Hexalone (1-(2,6,6-trimethyl-2-cyclohexen-1-yl)-1,6-heptadien-3-one), isocyclemone E (2-acetonaphthone-1,2,3,4,5,6,7,8-octahydro-2,3,8,8-tetramethyl), methylnonylketone, methylcyclocitrone, methyl lavender ketone, Orivone (4-tert-amyl-cyclohexanone), 4-tert-butylcyclohexanone, Delphone (2-pentylcyclopentanone), muscone (CAS 541-91-3), Neobutenone (1-(5,5-dimethyl-1-cyclohexenyl)pent-4-en-1-one), Plicatone (CAS 41724-19-0), Veloutone (2,2,5-trimethyl-5-pentylcyclopentan-1-one), 2,4,4,7-tetramethyl-oct-6-en-3-one, and tetramerane (6,10-dimethylundecen-2-one).

4. The composition according to one of claims 1 to 3, **characterized in that** the functional groups R² R⁴, R⁵ and R⁷ each represent hydrogen, and the functional group R⁶ represents a methyl, ethyl or hydroxymethyl functional group or hydrogen, and **in that** the functional groups R¹ and R³, independently of one another, each represent a C₆₋₂₄ hydrocarbon functional group, preferably a C₇₋₂₄ hydrocarbon functional group, the hydrocarbon functional group being able to be acyclic or cyclic, substituted or unsubstituted, branched or unbranched, and saturated or unsaturated.

5. The composition according to one of claims 1 to 4, **characterized in that** it contains further odorants.

6. The composition according to one of claims 1 to 5, **characterized in that** the components (a) and (b) are contained in amounts of from 0.1 to 99 wt.%, preferably from 1 to 50 wt.%, in particular from 5 to 30 wt.%, based on the total composition.

7. A perfumed consumer product, in particular a washing or cleaning agent, cosmetic agent, room-fragrancing agent or adhesives, **characterized in that** said consumer product contains a composition according to one of claims 1 to 6.

8. The washing or cleaning agent containing a composition according to one of claims 1 to 6 in amounts of from 0.0001 to 15 wt.%, based on the total agent.

9. A method for fragrancing textiles, **characterized in that** the textiles are subjected to a washing process using a composition according to one of claims 1 to 6 or a washing or cleaning agent according to claim 8.

10. The use of a perfume composition according to one of claims 1 to 6 in a washing or cleaning agent for prolonging the fragrance effect of the washing or cleaning agent and/or for achieving a long-lasting fresh scent when using the washing or cleaning agent.

## Revendications

1. Composition de parfum, comprenant
a) au moins un composant odorant aldéhyde de parfum avec au moins 6 atomes de carbone ou un composant odorant cétone avec au moins 6 atomes de carbone
ainsi que
b) au moins un précurseur de composant odorant oxazolidine correspondant au composant odorant aldéhyde ou au composant odorant cétone indiqués en a), lequel peut libérer le même composant odorant aldéhyde ou le même composant odorant cétone,
dans lequel le rapport molaire du composant odorant aldéhyde ou du composant odorant cétone a) sur le précurseur de composant odorant oxazolidine correspondant b) est de 20:1 à 1:20, de préférence de 10:1 à 1:10, avantageusement de 5:1 à 1:5 et
dans lequel il s'agit d'un précurseur de composant odorant oxazolidine sur un dérivé de 1-aza-3,7-dioxabicyclo[3.3.0]octane représenté par la formule générale (I) suivante 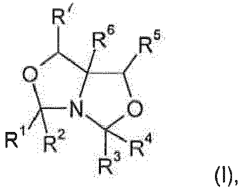 dans laquelle
R¹, R², R³, R⁴ indépendamment l'un de l'autre représentent pour les résidus, dans le dérivé de la formule générale R¹-C(=O)-R² ou R³-C(=O)-R⁴ un composant odorant aldéhyde avec au moins 6 atomes de carbone ou un composant odorant cétone avec au moins 6 atomes de carbone,
R⁵, R⁶, R⁷ indépendamment l'un de l'autre représentent H ou un résidu d'hydrocarbures qui peut être acyclique ou cyclique, substitué ou non substitué, non ramifié ou ramifié, saturé ou insaturé.

2. Composition selon la revendication 1, **caractérisée en ce que** le composant odorant aldéhyde est sélectionné parmi l'adoxal (2,6,10-triméthyl-9-undécénal), l'anisalaldéhyde (4-méthoxybenzaldéhyde), le cymal (3-(4-isopropylphényl)-2-méthylpropanal), l'éthylvanilline, le florhydral (3-(3-isopropylphényl)butanal), l'hélional (3-(3,4-méthylènedioxyphényl)-2-méthylpropanal), l'héliotropine, l'hydroxycitronellal, le laurylaldéhyde, le lyral (3- et 4-(4-hydroxy-4-méthylpentyl)-3-cyclohexène-1-carboxaldéhyde), le méthylnonylacétaldéhyde, le lilial (3-(4-tert-butylphényl)-2-méthylpropanal), le phénylacétaldéhyde, l'undécylénaldéhyde, la vanilline, le 2,6,10-triméthyl-9-undécénal, le 3-dodécen-1-al, l'alpha-n-amyl-amylzimt aldéhyde, le mélonal (2,6-diméthyl-5-hepténal), le 2,4-diméthyl-3-cyclohexène-1-carboxaldéhyde (triplal), le 4-méthoxy-benzaldéhyde, le benzaldéhyde, le 3-(4-tert-butylphényl)propanal, le 2-méthyl-3-(para-méthoxyphényl)propanal, le 2-méthyl-4- (2,6,6-triméthyl-2(1)cyclohexèn-1-yl)butanal, le 3-phényl-2-propénal, cis/trans-3,7-diméthyl-2,6-octadiène-1-al, le 3,7-diméthyl-6-octèn-1-al, le (3,7-diméthyl-6-octényl)oxy]acétaldéhyde, le 4-isopropylbenzylaldéhyde, le 1,2,3,4,5,6,7,8-octahydro-8,8-dimethyl-2-naphtaldéhyde, le 2,4-diméthyl-3-cyclohexène-1-carboxaldéhyde, le 2-méthyl-3-(isopropylphényl)propanal, le 1-décanal, le 2,6-diméthyl-5-hepténal, 4-(tricyclo[5.2.1.0(2,6)]-décylidène-8)-butanal, l'octahydro-4,7-méthano-1H-indencarboxaldéhyde, le 3-éthoxy-4-hydroxybenzaldéhyde, le para-éthyl-alpha,alpha-dimethylhydrozimtaldéhyde, l'alpha-méthyl-3,4-(méthylènedioxy-hydrozmitaldéhyde), le 3,4-méthylènedioxybenzaldéhyde, l'alpha-n-hexylzimtaldéhyde, le m-cymène-7-carboxaldéhyde, l'alpha-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényle)-3-cyclohexènecarboxaldéhyde, le 1-dodécanal, le 2,4-diméthylcyclohexène-3-carboxaldéhyde, le 4-(4-hydroxy-4-methylpentyl)-3-cylohexène-1-carboxaldéhyde, le 7-méthoxy-3,7-diméthyloctan-1-al, le 2-méthylundécanal, le 2-méthyldécanal, le 1-nonanal, le 1- octanal, le 2,6,10-triméthyl-5,9-undécadiénal, le 2-méthyl-3-(4-tert-butyl)propanal, le dihydrozimtaldéhyde, le 1-méthyl-4-(4-méthyl-3-pentényl)-3-cyclohexène-1-carboxaldéhyde, le 5- ou 6-méthoxyhexahydro-4,7-méthanindan-1- ou 2-carboxaldéhyde, le 3,7-diméthyloctan-1-al, le 1-undécanal, le 10-undécèn-1-al, le 4-hydroxy-3-méthoxy-benzaldéhyde, le 1-méthyl-3-(4-méthylpentyle)-3-cyclohexènecarboxaldéhyde, le 7-hydroxy-3,7-diméthyloctanal, le trans-4-décénal, le 2,6-nonadiénal, le para-tolylacétaldéhyde, le 4-méthylphénylacétaldéhyde, le 2-méthyl-4-(2,6,6-triméthyl-1-cyclohexèn-1-yl)-2-buténal, l'ortho-méthoxyzimtaldéhyde, le 3,5,6-triméthyl-3-cyclohexènecarboxaldéhyde, le 3,7-diméthyl-2-méthylène-6-octénal, le phénoxyacétaldéhyde, le 5,9-diméthyl-4,8-décadiénal, le päonienaldéhyde (6,10-diméthyl-3-oxa-5,9-undécadièn-1-al), l'hexahydro-4,7-méthanindan-1-carboxaldéhyde, le 2-méthyloctanal, l'alpha-méthyl-4-(1-méthyléthyl)benzolacétaldéhyde, le 6,6-diméthyl-2-norpinène-2-propionaldéhyde, le para-méthylphénoxyacétaldéhyde, le 2-méthyl-3-phényl-2-propén-1-al,, le 3,5,5-triméthylhexanal, l'hexahydro-8,8-diméthyl-2-naphtaldéhyde, le 3-propyl-bicyclo[2.2.1]-hept-5-én-2-carbaldéhyde, le 9-décénal, le 3-méthyl-5-phényl-1-pentanal, méthylnonylacétaldéhyde, l'hexanal et le trans-2-hexénal.

3. Composition selon la revendication 1 ou 2, **caractérisée en ce que** le composant odorant cétone est sélectionnée parmi la méthyl-bêta-naphtylcétone, le musc (1,2,3,5,6,7-hexahydro-1,1,2,3,3-pentaméthyl-4H-indén-4-one), la tonalide (6-acétyl-1,1,2,4,4,7-hexaméthyltétraline), l'alpha-damascone, la bêta-damascone, la delta-damascone, l'iso-damascone, la damascénone, le dihydrojasmonate de méthyle, la menthone, la carvone, le camphre, la koavone (3,4,5, 6,6-pentaméthylhept-3-én-2-one), la fenchone, l'alpha-ionone, la bêta-ionone, la gamma-méthylionone, la fleuramone (2-heptylcyclopentanone), la dihydrojasmone, la cis-jasmone, l'iso-E-super (1-(1,2,3,4,5,6,7,8-octahydro-2,3,8,8-tétraméthyl-2-naphtalényl)éthan-1-one (et isomères)), la méthylcédrénylcétone, l'acétophénone, la méthylacétophénone, la p-méthoxyacétophénone, la méthyl-bêta-naphtylcétone, la benzylacétone, la benzophénone, la para-hydroxyphénylbutanone, la cétone de céleri (3-méthyl-5-propyl-2-cyclohexénone), la 6-isopropyldécahydro-2-naphtone, la diméthylocténone, la frescomenthe (2-butan-2-yl-cyclohexan-1-one), la 4-(1-éthoxyvinyl)-3,3,5,5-tétraméthylcyclohexanone, la méthylhepténone, la 2-(2-(4-méthyl-3-cyclohexèn 1-yl)propyl)-cyclopentanone, la 1- (p-menthène-6(2)yl)-1-propanone, la 4-(4-hydroxy-3-méthoxyphényl)-2-butanone, le 2-acétyl-3, le 3-dimethylnorbornane, la 6,7-dihydro-1,1,2,3,3-pentaméthyl-4(5H)-indanone, le 4-damascol, le dulcinyle (4-(1,3-benzodioxol-5-yl)butan-2-one), l'hexalone (1-(2,6,6-triméthyl-2-cyclohexèn-1-yl)-1,6-heptadièn-3-one), l'isocyclémone E (2-acétonaphtone-1,2,3,4,5,6,7,8-octahydro-2,3,8,8-tétraméthyle), la méthylnonylcétone, la méthylcyclocitrone, la méthyl-lavande-cétone, l'orivone (4-tert-amyl-cyclohexanone), la 4-tert-butylcyclohexanone, la delphone (2-pentylcyclopentanone), la muscone (CAS 541-91-3), la néobuténone (1-(5,5-diméthyl-1-cyclohexényle)pent-4-én-1-one), la plicatone (CAS 41724-19-0), la veloutone (2,2,5-triméthyl-5-pentylcyclopentan-1-one), la 2,4,4,7-tétraméthyl-oct-6-én-3-one et la tétraméran(6,10-diméthylundécan-2-one).

4. Composition selon l'une quelconque des revendications 1 à 3, **caractérisée en ce que** les résidus R², R⁴, R⁵, R⁷ représentent chacun un atome d'hydrogène et R⁶ représente un résidu méthyle, éthyle ou hydroxyméthyle ou un atome d'hydrogène, et **en ce que** les résidus R¹ et R³, indépendamment l'un de l'autre représentent chacun un résidu d'hydrocarbyle en C₆₋₂₄, de préférence un résidu d'hydrocarbyle en C₇₋₂₄, dans lequel le résidu hydrocarboné peut être acyclique ou cyclique, substitué ou non substitué, ramifié ou non ramifié, ainsi que saturé ou insaturé.

5. Composition selon l'une quelconque des revendications 1 à 4, **caractérisée en ce qu'**elle contient d'autres composants odorants.

6. Composition selon une des revendications 1 à 5, **caractérisée en ce que** les composants (a) et (b) sont présents en des quantités de 0,1 à 99 % en poids, de préférence 1 à 50 % en poids, et en particulier de 5 à 30 % par rapport à la composition totale.

7. Produit de consommation parfumé, en particulier agent de lavage ou de nettoyage, agent cosmétique, ou agent parfumant d'ambiance ou adhésif, **caractérisé en ce qu'**il contient une composition selon l'une quelconque des revendications 1 à 6.

8. Agent de lavage ou de nettoyage comprenant une composition selon l'une quelconque des revendications 1 à 6 en des quantités de 0,0001 à 15 % en poids par rapport à l'agent global.

9. Procédé parfumant pour textiles, **caractérisé en ce que** l'on soumet le tissu à un traitement de lavage à l'aide d'une composition selon l'une quelconque des revendications 1 à 6 ou d'un agent de lavage ou de nettoyage selon la revendication 8.

10. Utilisation d'une composition de parfum selon l'une quelconque des revendications 1 à 6 dans un agent de lavage ou de nettoyage pour prolonger l'effet de parfum de l'agent de lavage ou de nettoyage et/ou pour obtenir une odeur fraîche de longue durée lors de l'application de l'agent de lavage ou de nettoyage.
